# FASCICULE DE BREVET EUROPEEN

(11) **EP 2 606 749 B1**
(45) Date de publication et mention de la délivrance du brevet: **16.04.2014**
(21) Numéro de dépôt: 12198797.8
(22) Date de dépôt: 21.12.2012
(51) Int. Cl.: A23L 1/302, A23L 1/305, A61K 35/36, A61K 36/06

(54) **Complément alimentaire pour le renforcement des racines capillaires et atténuer la chute des cheveux**
Nahrungsergänzungsmittel für die Stärkung der Haarwurzeln und zur Reduzierung von Haarausfall
Food supplement for strengthening hair roots and reducing hair loss

(30) Priorité: 23.12.2011 BE 201100733
(43) Date de publication de la demande: 26.06.2013
(73) Titulaire: S.A. Laboratoire Pharmaceutiques Trenker, 1180 Bruxelles (BE)
(72) Inventeur: Trenker, Rodolphe, 1470 Baisy-Thy (BE)
(74) Mandataire: Coulon, Ludivine

(56) Documents cités:
- WO-A1-2011/003015
- WO-A1-2011/115227
- FR-A5- 2 044 595
- JP-A- 2004 357 508
- JP-A- 2007 236 383

## Description

La présente invention se rapporte à un complément alimentaire comprenant de la kératine hydrosoluble pour renforcer les racines capillaires et atténuer la perte des cheveux.

La perte et la repousse des cheveux sont des phénomènes normaux qui se compensent lorsque l'équilibre naturel des cheveux est assuré. Quotidiennement, une cinquantaine de cheveux meurent et tombent, ce qui n'a pas de conséquence particulière en raison de l'activité pilaire cyclique. Cependant, une perte excessive et irréversible des cheveux peut avoir lieu selon des processus locaux ou systémiques qui perturbent l'équilibre entre la perte et la repousse des cheveux. Parmi les facteurs de déséquilibre systémique, le stress, la fatigue et le statut hormonal sont les plus fréquents. Par exemple, une hypothyroïdie peut entraîner une chute diffuse des cheveux qui sont ternes et fragiles ou peut provoquer une alopécie. Parmi les facteurs locaux, l'utilisation quotidienne de produits coiffants et l'usage de sèche-cheveux peuvent modifier l'équilibre naturel de la chevelure.

Il est connu de l'état de la technique que la kératine, certaines vitamines et certains minéraux contribuent largement à l'équilibre naturel de la chevelure.

La kératine est une protéine fibreuse insoluble qui contient une forte proportion d'acides aminés soufrés, principalement de la cystéine. Cette forte proportion de cystéine donne lieu à la formation de ponts disulfures entre acides aminés pour former des dimères de cystéines ou cystines. Ces acides aminés dipeptidiques composés de deux acides aminés cystéine joints par des ponts disulfures sont à la base de la structure et de la rigidité des cheveux. Les acides aminés soufrés ne pouvant pas être bio-synthétisés de novo, il est nécessaire de les absorber via l'alimentation. Cependant, les cystéines sont peu bio-disponibles lors de l'absorption de kératine à cause des ponts disulfures particulièrement stables. De plus, la kératine résiste aux digestions enzymatiques par la pepsine et la trypsine, ce qui la rend d'autant plus difficile à absorber. Il est donc indispensable d'absorber la kératine sous une forme bio-disponible.

Les vitamines B ont des effets divers, directs et indirects, sur les cheveux. Par exemple, il est connu que la vitamine B2 (riboflavine) protège les cellules capillaires des dommages oxydatifs. La vitamine B3 (niacine) contribue à l'équilibre naturel des cheveux. Les vitamines B5 (acide pantothénique) et B8 (D-biotine) ont une action stimulante sur le follicule pileux et la régulation de la séborrhée. La vitamine B6 (chlorhydrate de pyridoxine) est une coenzyme indispensable à la synthèse de la kératine et contribue à l'équilibre naturel des cheveux. La vitamine B9 (acide folique) intervient dans le métabolisme des acides aminés.

D'autres vitamines sont également connues pour avoir un effet sur les cheveux. Ainsi, la vitamine A (bétacarotène) prévient le vieillissement précoce des cheveux et contribue à la protection contre les radicaux libres. La vitamine C (L-ascorbate de sodium) fortifie les cheveux et est indispensable à la synthèse de la kératine. La vitamine E (hydrogénosuccinate de d-a-tocophéryle) prévient également le vieillissement précoce des cheveux, contribue à la protection contre les radicaux libres et favorise l'oxygénation et la circulation capillaire.

Des minéraux tels que le fer, le cuivre et le zinc sont également bénéfiques pour les cheveux. Ces trois minéraux inhibent faiblement la 5-a-réductase de type I, une enzyme qui catalyse la réduction de la testostérone en dihydrotestostérone étant responsable de l'alopécie androgénique (affection héréditaire qui consiste en une perte de cheveux induite par les androgènes). De plus, le fer intervient dans la nutrition et l'oxygénation de la racine des cheveux tandis que le zinc réduit la production de sébum et consolide la structure des cheveux. Le cuivre contribue à l'absorption et à l'utilisation du fer par les racines des cheveux.

L'utilisation de kératine pour le soin des cheveux est connue. Par exemple, le brevet FR2438662 divulgue l'utilisation d'un hydrolysat hydrosoluble de kératine utile comme produit cosmétique capillaire et son procédé de préparation, ce produit étant appliqué sur les cheveux pour obtenir un effet d'ondulation ou de fixation des cheveux sans les endommager.

Par ailleurs, des compléments alimentaires comprenant de la kératine et certaines vitamines et/ou minéraux sont connus de l'état de la technique. Par exemple, le document W02011/003015 divulgue un complément alimentaire bénéfique pour la santé comprenant de la kératine solubilisée en association avec des vitamines et/ou des minéraux.

Un autre complément alimentaire (pour animaux) est décrit dans FR 2 044 595.

Les vitamines généralement associées à la kératine sont les vitamines C, B3, B5, B6 et B8.

Malheureusement, la formulation de tels complément alimentaire est difficile car certaines vitamines et/ou minéraux doivent être présents dans la composition finale seulement à l'état de trace (quelques mg voire quelques µg) alors que d'autres constituants tels que la kératine soluble sont ajoutés en des quantités largement supérieures, ce qui rend l'homogénéité de la composition finale extrêmement difficile à obtenir.

Pour résoudre ce problème d'homogénéisation de la composition finale, il est prévu suivant l'invention, un complément alimentaire tel qu'indiqué au début qui comprend une levure enrichie en un complexe de vitamines B et désactivée, ledit complexe de vitamines B comprenant au moins sept vitamines B différentes.

Par les termes « complexe de vitamines B » au sens de la présente invention, on entend un ensemble de vitamines B hydrosolubles incorporées dans une matrice organique en des proportions définies.

L'intégration d'une levure dans la composition permet d'alléger le procédé de fabrication du complément alimentaire et d'assurer une homogénéisation de la composition finale dudit complément alimentaire. En effet, les apports journaliers en vitamines B et/ou en minéraux correspondant à des quantités très faibles de l'ordre du mg voire du µg, la mise en oeuvre de ces quantités dans des formulations complètes comprenant d'autres substances que des vitamines B et/ou des minéraux est difficile. Il est en effet difficile d'assurer une homogénéité correcte du mélange lorsque les quantités peuvent varier d'un rapport 1 à 1000 entre les différents constituants, ces quantités étant imposées par la formulation, par exemple pour que les AJR en chaque constituant soient assurés. Avec de telles dispersions de masses, il est nécessaire de procéder à plusieurs triturations successives afin d'aboutir à une homogénéité correcte des poudres. Ces opérations de mélange sont longues et doivent être contrôlées, ce qui constitue une étape supplémentaire et coûteuse lors de la production. De telles mises en oeuvre ne sont plus nécessaires lors de l'ajout des vitamines B à la composition par l'intermédiaire d'une levure enrichie en un complexe d'au moins sept vitamines B puis désactivée, ladite levure comprenant chaque vitamine en une proportion déterminée et recherchée. Il est en effet possible de faire se développer des levures sur des milieux de culture adaptés de manière prédéterminée pour influencer la teneur en vitamines B de ces levures et ce de manière reproductible. Ainsi, il est possible et particulièrement avantageux d'ajouter la levure enrichie puis désactivée en une quantité en poids relativement similaire à la quantité en poids de kératine hydrosoluble, ce qui permet d'aboutir à une homogénéisation correcte des poudres et donc à une composition selon l'invention comprenant chaque constituant, dont les vitamines, en des proportions adéquates prédéterminées. De préférence, un rapport en poids kératine hydrosoluble/ levure enrichie puis désactivée de 1/1 ou proche de 1/1 sera recherché.

Avantageusement, le complément alimentaire comprend de la kératine hydrosoluble (hydrolysat soluble de kératine) comprenant les précurseurs de la kératine qui fournissent sous une forme bio-disponible les acides aminés soufrés indispensables à la synthèse de la kératine. Les acides aminés soufrés n'étant pas bio-synthétisable de novo, l'ingestion des précurseurs de la kératine par l'intermédiaire d'un complément alimentaire est indispensable d'autant plus que la kératine en elle-même n'est pas assimilable à cause de sa résistance aux digestions enzymatiques. L'absorption d'un tel hydrolysat de kératine contenant au moins 60% de peptides (protéines) contribue à la santé et à la vitalité des cheveux en en augmentant la brillance, en en renforçant le maintien et en en minimisant la chute. Par exemple, la kératine hydrosoluble peut être intégrée dans le complément alimentaire sous forme d'une poudre soluble de kératine telle que la Cynatine ® HNS.

De préférence, selon la présente invention, la levure enrichie en vitamines B est une levure autorisée dans le domaine alimentaire et connue pour ses effets positifs contribuant à l'équilibre général de la chevelure, telle que par exemple la levure Saccharomyces cerevisiae. Une telle levure est particulièrement avantageuse puisqu'elle constitue une source naturelle de vitamines B et qu'elle peut être enrichie en vitamines B afin de définir une quantité déterminée pour chacune des vitamines B. Il est en effet possible de faire se développer des levures sur des milieux de culture adaptés de manière prédéterminée pour influencer la teneur en vitamines B de ces levures et ce de manière reproductible. Par exemple, une levure peut être enrichie en vitamines B puis désactivée pour assurer la couverture quasi complète des apports journaliers recommandés en les vitamines B1, B2, B3, B5, B6, B8 et B9 : 1,1 mg de vitamine B1, 1,4 de vitamine B2, 16 mg de vitamine B3, 6 mg de vitamine B5, de 1,4 mg de vitamine B6, de 50 µg de vitamine B8 et 200 µg de vitamine B9. Selon la présente invention, les quantités apportées en les vitamines B1, B2, B3, B5, B6, B8 et B9 sont de 0,9 à 1,3 mg de vitamine B1, de 1,3 à 1,9 mg de vitamine B2, de 13 à 19 mg de vitamine B3, de 4,8 à 6,6 mg de vitamine B5, de 1,6 à 2,4 mg de vitamine B6, de 25 à 80 µg de vitamine B8 et de 190 à 310 µg de vitamine B9.

Avantageusement, le complément alimentaire comprend au moins une vitamine additionnelle choisie dans le groupe constitué de la vitamine A, de la vitamine C et de la vitamine E ainsi que de leur précurseurs. Pour assurer les AJR en lesdites vitamines additionnelles, le complément alimentaire comprend ainsi de préférence 120 à 1200 µg de vitamine A et/ou de 9 à 180 mg de vitamine C et/ou de 1,5 à 30 mg de vitamine E, ces vitamines ayant un effet bénéfique pour l'équilibre naturel de la chevelure (protection contre les radicaux libres, prévention du vieillissement précoce du cheveu, ...).

De façon tout aussi avantageuse, le complément alimentaire comprend au moins un minéral choisi dans le groupe constitué du Fe, du Cu et du Zn, ledit au moins un minéral ayant également un effet positif pour l'équilibre naturel de la chevelure en prévenant notamment la perte de cheveux induite par un excès de dihydrotestostérone.

De préférence, pour que ledit au moins un minéral soit toléré correctement et absorbé de manière à procurer les AJR, le complément alimentaire comprend de préférence 2,1 à 28 mg de Fe, 2,25 à 22,5 mg de Zn et 0,165 à 1,65 mg de Cu sous forme de gluconate plutôt que sous forme d'oxydes de Fe, de Zn ou de Cu. Ces quantités de minéraux correspondent à 16,8 à 223,7 mg de gluconate de Fe, 15,7 à 156,8 mg de gluconate de Zn et 1,18 à 117,8 mg de gluconate de Cu. Les gluconates sont généralement des additifs alimentaires jouant le rôle de séquestrant, ce qui améliore notamment la qualité et la stabilité de produits alimentaires tout en facilitant leur absorption.

Avantageusement, le complément alimentaire comprend la cellulose microcristalline et la silice colloïdale comme excipients. La cellulose microcristalline est généralement obtenue sous forme de pulpe à partir de matière végétale fibreuse naturelle tandis que la silice colloïdale est une poudre naturelle de quartz extrêmement fine. Lors du conditionnement du complément alimentaire par exemple sous forme de gélule, la cellulose microcristalline permet le remplissage de la gélule et la fixation d'un poids déterminé tandis que la silice colloïdale améliore la fluidité de la poudre lors de la mise en gélule.

Avantageusement, le complément alimentaire est présenté sous forme d'un sirop ou d'une lotion ou d'un shampoing.

Préférentiellement, le complément alimentaire est présenté sous forme d'une gélule formée au départ d'un polymère d'origine végétale, de préférence l'hydroxypropylméthylcellulose ou hypromellose.

De façon avantageuse, la gélule contenant le complément alimentaire est colorée à l'oxyde de fer rouge plutôt qu'avec du dioxyde de titane pour des raisons d'innocuité de ces substances.

Préférentiellement, le complément alimentaire selon la présente invention est administré par la prise d'une ou de deux gélules, selon les besoins de l'utilisateur. La prise d'une gélule est destinée à un entretien de la chevelure tandis que la prise de deux gélules est à envisager lors d'une chute anormale des cheveux.

Selon la présente invention, la prise de deux gélules, ce qui correspond à la prise de 500 mg de kératine soluble et de 500 mg d'une levure enrichie en un complexe de vitamines B et désactivée, assure simultanément les AJR en vitamines A, B1, B2, B3, B5, B6, B8, B9, C et E mais aussi en les minéraux Zn, Cu et Fe.

La présente invention va être maintenant décrite à l'aide d'un exemple de réalisation. Il va de soi que cet exemple n'est en aucune façon limitatif.

### Exemple : apports en vitamines et en minéraux lors de la prise du complément alimentaire

De la poudre soluble de kératine Cynatine ® HNS (hydrolysat soluble de kératine) disponible auprès de la société Roxlor, de la levure FortifYeat B-Complex ® (Saccharomyces cerevisiae) disponible auprès de la société Lallemand, des vitamines A, C et E, du gluconate de Fe, du gluconate de Zn, du gluconate de Cu sont mélangés selon les quantités reprises dans le tableau afin de rencontrer les besoins physiologiques en les divers constituants. De la silice colloïdale et de la cellulose microcristalline sont ajoutées comme excipients à raison de 4,2 mg et 1400 mg respectivement.

**Tableau : quantités apportées en vitamines et en minéraux**

| | **Masse** | **AJR (%)** |
|---|---|---|
| Cynatine ® HNS | 500 mg | / |
| FortifYeat B-Complex ® | 500 mg | / |
| Vitamine B1 | 1,1 mg | 100 |
| Vitamine B2 | 1,4 mg | 100 |
| Vitamine B3 | 16 mg | 100 |
| Vitamine B5 | 6 mg | 100 |
| Vitamine B6 | 1,4 mg | 100 |
| Vitamine B8 | 50 µg | 100 |
| Vitamine B9 | 200 µg | 100 |
| Gluconate de Zn | 75,52 mg | 100 (Zn) |
| Gluconate de Cu | 7,14 mg | 100 (Cu) |
| Gluconate de Fe | 114,72 mg | 100 % (Fer) |
| Vitamine A (bétacarotène à 10%) | 48 mg | 100 |
| Vitamine C | 80 mg | 100 |
| Vitamine E | 14,80 mg | 100 |

## Revendications

1. Complément alimentaire comprenant de la kératine hydrosoluble pour renforcer les racines capillaires et atténuer la perte des cheveux, **caractérisé en ce qu'**il comprend en outre une levure enrichie en un complexe de vitamines B et désactivée, ledit complexe de vitamines B comprenant au moins sept vitamines B différentes.

2. Complément alimentaire selon la revendication 1, **caractérisé en ce que** ladite levure est Saccharomyces cerevisiae.

3. Complément alimentaire selon les revendications 1 ou 2, **caractérisé en ce que** ledit complexe de vitamines B comprend au moins les vitamines B1, B2, B3, B5, B6, B8, et B9.

4. Complément alimentaire selon l'une des quelconques revendications 1 à 3, **caractérisé en ce qu'**il comprend en outre au moins une vitamine additionnelle choisie dans le groupe constitué des vitamine A, vitamine C et vitamine E ainsi que de leur précurseurs.

5. Complément alimentaire selon l'une des quelconques revendications 1 à 4, **caractérisé en ce qu'**il comprend en outre au moins un minéral choisi dans le groupe constitué du Fe, du Cu et du Zn.

6. Complément alimentaire selon la revendication 5, **caractérisé en ce que** lesdits minéraux sont sous la forme de gluconates.

7. Complément alimentaire selon l'une des quelconques revendications 1 à 6, **caractérisé en ce qu'**il comprend un excipient, de préférence de la silice colloïdale et de la cellulose microcristalline.

8. Complément alimentaire selon l'une des quelconques revendications 1 à 7, **caractérisé en ce qu'**il est conditionné préférentiellement sous forme d'une gélule, d'une lotion, d'un sirop, d'un onguent, d'un masque ou d'un shampoing.

9. Complément alimentaire selon la revendication 8, **caractérisé en ce que** ladite gélule est formée au départ d'un polymère d'origine végétale.

10. Complément alimentaire selon la revendication 9, **caractérisé en ce que** ledit polymère d'origine végétale est l'hydroxypropylméthylcellulose.

11. Complément alimentaire selon la revendication 9, caractérisé en ce ladite gélule est colorée à l'oxyde de fer rouge.

## Patentansprüche

1. Nahrungsergänzungsmittel, das wasserlösliches Keratin umfasst zum Stärken der Haarwurzeln und zum Vermindern von Haarausfall, **dadurch gekennzeichnet, dass** es ferner eine Hefe umfasst, die mit einem Komplex von B-Vitaminen angereichert und deaktiviert ist, wobei der Komplex von B-Vitaminen mindestens sieben verschiedene B-Vitamine umfasst.

2. Nahrungsergänzungsmittel nach Anspruch 1, **dadurch gekennzeichnet, dass** die Hefe Saccharomyces cerevisiae ist.

3. Nahrungsergänzungsmittel nach Anspruch 1 oder 2, **dadurch gekennzeichnet, dass** der Komplex von B-Vitaminen mindestens die Vitamine B1, B2, B3, B5, B6, B8 und B9 umfasst.

4. Nahrungsergänzungsmittel nach einem der Ansprüche 1 bis 3, **dadurch gekennzeichnet, dass** es ferner mindestens ein weiteres Vitamin umfasst, das ausgewählt ist aus der Gruppe bestehend aus Vitamin A, Vitamin C und Vitamin E sowie aus deren Vorläufern.

5. Nahrungsergänzungsmittel nach einem der Ansprüche 1 bis 4, **dadurch gekennzeichnet, dass** es ferner mindestens ein Mineral umfasst, das ausgewählt ist aus der Gruppe bestehend aus Fe, Cu und Zn.

6. Nahrungsergänzungsmittel nach Anspruch 5, **dadurch gekennzeichnet, dass** die Mineralien in Form von Gluconaten vorliegen.

7. Nahrungsergänzungsmittel nach einem der Ansprüche 1 bis 6, **dadurch gekennzeichnet, dass** es einen Trägerstoff, vorzugsweise kolloidale Kieselsäure und mikrokristalline Cellulose umfasst.

8. Nahrungsergänzungsmittel nach einem der Ansprüche 1 bis 7, **dadurch gekennzeichnet, dass** es vorzugsweise in Form einer Kapsel, einer Lotion, eines Sirups, einer Salbe, einer Maske oder eines Shampoos abgepackt ist.

9. Nahrungsergänzungsmittel nach Anspruch 8, **dadurch gekennzeichnet, dass** die Kapsel aus einem Polymer pflanzlichen Ursprungs gebildet wird.

10. Nahrungsergänzungsmittel nach Anspruch 9, **dadurch gekennzeichnet, dass** das Polymer pflanzlichen Ursprungs Hydroxypropylmethylcellulose ist.

11. Nahrungsergänzungsmittel nach Anspruch 9, **dadurch gekennzeichnet, dass** die Kapsel mit rotem Eisenoxid gefärbt ist.

## Claims

1. A food supplement comprising water-soluble keratin for reinforcing capillary roots and attenuating hair loss, **characterized in that** it further comprises a yeast enriched with a complex of vitamins B and deactivated, said complex of vitamins B comprising at least seven different vitamins B.

2. The food supplement according to claim 1, **characterized in that** said yeast is *Saccharomyces cerevisiae.*

3. The food supplement according to claims 1 or 2, **characterized in that** said complex of vitamins B comprises at least the vitamins B1, B2, B3, B5, B6, B8, and B9.

4. The food supplement according to one of any claims 1 to 3, **characterized in that** it further comprises at least one additional vitamin selected from the group consisting of vitamin A, vitamin C and vitamin E as well as their precursors.

5. The food supplement according to one of any claims 1 to 4, **characterized in that** it further comprises at least one mineral selected from the group consisting of Fe, Cu and Zn.

6. The food supplement according to claim 5, **characterized in that** said minerals are in the form of gluconates.

7. The food supplement according to one of any claims 1 to 6, **characterized in that** it comprises an excipient, preferably colloidal silica and microcrystalline cellulose.

8. The food supplement according to one of any claims 1 to 7, **characterized in that** it is preferentially conditioned as a gelatin capsule, a lotion, a syrup, an ointment, a mask or a shampoo.

9. The food supplement according to claim 8, **characterized in that** said gelatin capsule is formed, starting with a polymer of plant origin.

10. The food supplement according to claim 9, **characterized in that** said polymer of plant origin is hydroxypropylmethylcellose.

11. The food supplement according to claim 9, **characterized in that** said gelatin capsule is colored with red iron oxide.
